# EUROPÄISCHE PATENTSCHRIFT

(11) **EP 2 352 454 B1**
(45) Veröffentlichungstag und Bekanntmachung des Hinweises auf die Patenterteilung: **15.02.2017**
(21) Anmeldenummer: 09778127.2
(22) Anmeldetag: 26.08.2009
(51) Int. Cl.: A61B 18/16, A61B 18/00

(54) **NEUTRALELEKTRODENVORRICHTUNG ZUR KÜHLUNG EINER ELEKTRODE**
NEUTRAL ELECTRODE DEVICE FOR COOLING AN ELECTRODE
SYSTÈME D'ÉLECTRODE NEUTRE POUR LE REFROIDISSEMENT D'UNE ÉLECTRODE

(30) Priorität: 09.09.2008 DE 102008046300
(43) Veröffentlichungstag der Anmeldung: 10.08.2011
(73) Patentinhaber: Erbe Elektromedizin GmbH, 72072 Tübingen (DE)
(72) Erfinder: HAGG, Martin, 72827 Wannweil (DE); SELIG, Peter, 72379 Hechingen (DE)
(74) Vertreter: Bohnenberger, Johannes
(86) Internationale Anmeldenummer: PCT/EP2009/006186
(87) Internationale Veröffentlichungsnummer: WO 2010/028750

(56) Entgegenhaltungen:
- EP-A- 1 905 372
- EP-A- 1 905 374
- WO-A-02/060526
- US-A- 4 343 308
- US-A- 4 387 714
- US-A1- 2002 198 523

## Beschreibung

Die Erfindung betrifft eine Neutralelektrodenvorrichtung mit einem Latentwärmespeicher, ein elektrochirurgisches Instrument mit entsprechender Elektrodenvorrichtung, ein Kontaktmittel mit Latentwärmespeicher und die Verwendung eines Latentwärmespeichers zur Kühlung einer Elektrode.

Bei der Hochfrequenzchirurgie (HF-Chirurgie) wird Wechselstrom mit hoher Frequenz durch den menschlichen Körper geleitet, um Gewebe gezielt zu schädigen bzw. zu schneiden. Ein wesentlicher Vorteil gegenüber herkömmlichen Schneidetechniken mit dem Skalpell ist, dass gleichzeitig mit dem Schnitt eine Blutungsstillung durch Verschluss der betreffenden Gefäße erfolgen kann.

Häufig wird eine monopolare Technik angewandt. Dabei wird ein Pol der HF-Spannungsquelle über eine möglichst große Fläche mit dem Patienten verbunden. Diese Elektrode nennt man Neutralelektrode. Der andere Pol (Aktivelektrode) befindet sich an einem chirurgischen Instrument. Der Strom fließt von der Aktivelektrode zur Neutralelektrode. In unmittelbarer Nähe der Aktivelektrode ist die Stromdichte am höchsten, hier findet eine Koagulation oder Durchtrennung des Gewebes statt.

Bei Neutralelektroden muss darauf geachtet werden, dass zwischen Haut und anliegender Elektrode kein zu hoher Übergangswiderstand auftritt. Dies würde zu einer starken Erwärmung des biologischen Gewebes, mitunter zu Verbrennungen führen. In letzter Zeit stellt sich das Problem, dass immer mehr Verfahren entwickelt wurden, bei denen relativ große HF-Ströme über längere Zeitdauer appliziert werden. Das Risiko einer Verbrennung an der Neutralelektrode wird somit erhöht. Hierbei sei noch angemerkt, dass durch die physikalischen Gegebenheiten an den Randbereichen der Neutralelektrode die maximale Erwärmung auftritt. Daher ist in diesen Randbereichen das Risiko einer Verbrennung besonders hoch.

Um eine ungewollte Schädigung des Gewebes zu vermeiden, werden entsprechend großflächige Neutralelektroden verwendet, die dazu beitragen, die Stromdichte in unmittelbarer Nachbarschaft der Neutralelektrode zu verringern. Darüber hinaus gibt es Überwachungseinrichtungen, die den Zweck haben, ein teilweises Ablösen der Neutralelektrode zu erkennen und auf dieses Ereignis entsprechend zu reagieren.

Aktuelle Normen schreiben Untersuchungen vor, die einen Temperaturanstieg an der Neutralelektrode bei der Applikation eines gewissen Stroms über einen vorgegebenen Zeitraum auf einen Maximalwert begrenzen.

Bei den herkömmlich gewählten Lösungsansätzen besteht ein weiteres Problem darin, dass die Fläche der Neutralelektrode nicht beliebig vergrößert werden kann, da die Applikation ansonsten nicht mehr praktikabel ist. Ein geeignetes Anbringen der Neutralelektrode wird mit zunehmender Größe schwieriger. Des Weiteren sind bei Anwendungen in der Kinderchirurgie enge Grenzen bezüglich der Größe der Elektrode gesetzt.

Die heute bekannten und verwendeten Überwachungseinrichtungen können nur auf indirekte Weise den Gefährdungsgrad bestimmen, da meist lediglich der elektrische Übergangswiderstand zwischen Neutralelektrode und Patienten gemessen wird, was nur vage in Korrelation zu der Anlagefläche steht.

Wie oben bereits erwähnt, sind die Stromdichten auf der Neutralelektrode nicht gleichmäßig verteilt. So kann es beispielsweise an den Rändern zu einer starken Erwärmung kommen, die zu Verbrennungen führt. Diese lokalen Effekte zu überwachen, erweist sich als extrem schwierig.

Ausgehend von diesem Stand der Technik ist es Aufgabe der vorliegenden Erfindung, eine verbesserte Neutralelektrodenvorrichtung bereit zu stellen. Insbesondere soll eine Schädigung des Gewebes durch den HF-Strom im Bereich der Neutralelektrode vermieden werden. Des Weiteren soll ein entsprechend verbessertes elektrochirurgisches Instrument und ein Kontaktmittel angegeben werden.

Diese Aufgabe wird durch eine Neutralelektrodenvorrichtung nach Anspruch 1

Weitere vorteilhafte Ausführungsformen ergeben sich aus den Unteransprüchen.

Insbesondere wird die Aufgabe durch eine Neutralelektrodenvorrichtung zur Applikation eines HF-Stroms an einem biologischen Gewebe gelöst, wobei die Vorrichtung mindestens einen Latentwärmespeicher zur Aufnahme von Wärme umfasst.

Der Kern der Erfindung besteht also darin, dass einer Verbrennung aktiv entgegen gewirkt wird, indem ein Temperaturanstieg in einen kritischen Bereich durch Kühleffekte ausreichend verhindert wird. Die erfindungsgemäße Neutralelektrodenvorrichtung umfasst hierfür einen Latentwärmespeicher, der die thermischen Spitzen, die bei einer Behandlung auftreten, aufnimmt und über einen langen Zeitraum speichert. Somit ist es möglich, kurzfristig auftretende Temperaturerhöhungen abzufangen. Die gespeicherte thermische Energie kann entweder im Verlauf der Operation oder nach der Operation abgegeben werden. Damit kann in die Neutralelektrodenanordnung eine thermische Sicherheitsreserve eingebaut werden, die auch bei längeren Aktivierungszyklen mit größeren Strömen eine kritische Temperaturerhöhung effektiv verhindert.

Die Neutralelektrodenvorrichtung kann mindestens eine Elektrode, insbesondere aus Aluminium umfassen, wobei der Latentwärmespeicher flächig auf der Elektrode angeordnet ist. Es ist vorteilhaft, wenn der Latentwärmespeicher zur Aufnahme von Wärmeenergie über die gesamte Elektrodenoberfläche verteilt ist. So kann die Wärmeenergie stets dort aufgenommen werden, wo sie entsteht. Denkbar wäre auch eine an die auftretende Wärmeverteilung angepasste Verteilung des Latentwärmespeichers. Beispielsweise könnte ein leistungsstärkerer Latentwärmespeicher an den Rändern der Elektrode vorgesehen werden.

Der Latentwärmespeicher kann bei der Applikation auf einer dem biologischen Gewebe abgewandten Seite der Elektrode angeordnet sein. Der Latentwärmespeicher stört somit die Applikation des HF-Stroms nicht. Insbesondere wirkt er nicht als Widerstand, der zu einer ungewollten Temperaturerhöhung der Neutralelektrodenvorrichtung führen kann. Des Weiteren wird eine direkte Kontaktierung des Latentwärmespeichers mit dem biologischen Gewebe vermieden. Auch können mögliche Kompatibilitätsprobleme mit dem bei der Applikation verwendeten Hydrogel vermieden werden. Eine sichere Aufnahme der Wärmeenergie kann durch eine unmittelbare Kontaktierung der Elektrode erzielt werden.

Die Neutralelektrodenvorrichtung kann mindestens eine Trägerfaserschicht mit Phasenübergangsmaterial (PCM: Phase Change Materials) umfassen. Es ist üblich, die Elektroden der Neutralelektrodenvorrichtung auf ein flexibles Gewebe oder Trägervlies aufzubringen, um eine optimale Kontaktierung mit den individuellen anatomischen Strukturen des Patienten zu ermöglichen. Das Phasenübergangsmaterial kann dieses Trägervlies ergänzen oder ersetzen. Beispielsweise können PCM-Fasern in dem Trägervlies verarbeitet werden. Alternativ kann das Trägervlies durch PCM-Fasern ersetzt werden.

Alternativ oder zusätzlich kann die Neutralelektrodenvorrichtung einen ein Kühlkissen umfassenden Latentwärmespeicher aufweisen. Das Kühlkissen ließe sich beispielsweise auf die eigentliche Neutralelektrodenvorrichtung auflegen. Somit wären große Materialmengen des Latentwärmespeichers realisierbar. Des Weiteren könnte ein entsprechendes Kühlkissen wiederverwendet werden. Die Handhabung des Kühlkissens ist denkbar einfach. Es kann während der Operation ausgewechselt werden. Soweit es während der Operation zu einer derartigen Überhitzung der Elektroden kommen würde, dass die Kapazitäten des Latentwärmespeichers aufgebraucht sind, wäre ein Austausch ohne großen Aufwand möglich.

Des Weiteren wird die Aufgabe durch ein elektrochirurgisches Instrument zur Koagulation und/oder zum Schneiden von Gewebe gelöst, wobei das Instrument eine Neutralelektrodenvorrichtung wie vorab beschrieben umfasst.
Es ergeben sich dieselben Vorteile.

Auch kann die Aufgabe durch ein Kontaktmittel zur Verbesserung des elektrischen Kontaktschlusses zwischen einer Elektrode und einem biologischen Gewebe gelöst werden, wobei das Kontaktmittel mindestens einen Latentwärmespeicher zur Aufnahme von Wärme und eine leitende Substanz umfasst. Es ist also möglich, ein Kontaktmittel für Neutralelektroden bereit zu stellen, das den Kontaktschluss zwischen der Elektrode und dem biologischen Gewebe verbessert, wobei das Kontaktmittel des Weiteren einen Wärmelatentspeicher aufweist, der dazu geeignet ist, die auftretende Wärme zu absorbieren. Ein besonderer Vorteil des Kontaktmittels besteht darin, dass der Latentwärmespeicher die thermische Energie von Elektrode und elektrischem Gewebe aufnehmen kann. Eine Anpassung der Neutralelektrode entfällt.

Die leitende Substanz kann aus der Gruppe der viskoelastischen Fluide sein. Insbesondere kann die leitende Substanz ein Hydrogel sein. Hydrogel ist besonders dazu geeignet, den elektrischen Kontaktschluss zwischen Elektroden und biologischem Gewebe zu verbessern. Des Weiteren lässt sich das Hydrogel leicht applizieren. Das Hydrogel kann mit Phasenübergangsmaterial, z.B. in Pulverform durchmischt werden. Ebenfalls denkbar wäre ein zweischichtiges Gel, bei dem die untere Schicht, die in Kontakt mit dem biologischen Gewebe steht, die leitende Substanz oder das Hydrogel, und die obere Schicht ein Phasenübergangsmaterial in Gelform ist.

Des Weiteren wird die Aufgabe durch die Verwendung eines Latentwärmespeichers zur Kühlung einer Neutralelektrode, insbesondere für HF-chirurgische Anwendungen, gelöst.

Auch hier ergeben sich ähnliche Vorteile wie vorab beschrieben.

Der oben beschriebene Latentwärmespeicher kann ein Phasenübergangsmaterial, insbesondere aus der Stoffgruppe der Paraffine sein.

Zur besseren Verarbeitung kann das Phasenübergangsmaterial in Silikat oder Kunstfasern gekapselt sein.

Der Latentwärmespeicher kann einen Schmelzpunkt aufweisen, der niedriger als eine Maximaltemperatur ist, bei der es zur thermischen Beschädigung von biologischem Gewebe kommt. Somit wird der Latentwärmespeicher erst dann aktiviert, wenn sich das biologische Gewebe oder die Neutralelektrodenanordnung einer kritischen Temperatur annähert. Die Ressourcen des Latentwärmespeichers können somit optimal ausgenutzt werden.

Die Maximaltemperatur kann niedriger als 70°C, insbesondere niedriger als 60°C, insbesondere niedriger als 50°C, insbesondere niedriger als 40°C, insbesondere niedriger als 35°C, insbesondere niedriger als 30°C sein.

Sinnvollerweise sollte der Schmelzpunkt des eingesetzten Materials so gewählt werden, dass er höher als die Oberflächentemperatur des biologischen Gewebes ist. Insbesondere sollte der Schmelzpunkt höher als eine Minimaltemperatur, insbesondere höher als 20°C, insbesondere höher als 25°C sein.

Nachfolgend wird die Erfindung anhand von einigen Ausführungsbeispielen beschrieben, die mittels Abbildungen näher erläutert werden. Hierbei zeigen:
- - Fig. 1: ein monopolares elektrochirurgisches Instrument zum Schneiden und/oder Koagulieren von Gewebe;
- - Fig. 2: eine Neutralelektrode mit Hydrogel, wobei das Hydrogel mit PCM versetzt ist;
- - Fig. 3: eine Neutralelektrode mit einer zusätzlichen PCM-Schicht;
- - Fig. 4: eine Neutralelektrode mit einem Trägervlies aus PCM-Fasern; und
- - Fig. 5: eine Neutralelektrode mit einem PCM-Kissen.

In der nachfolgenden Beschreibung werden für gleiche und gleich wirkende Teile dieselben Bezugsziffern verwendet.

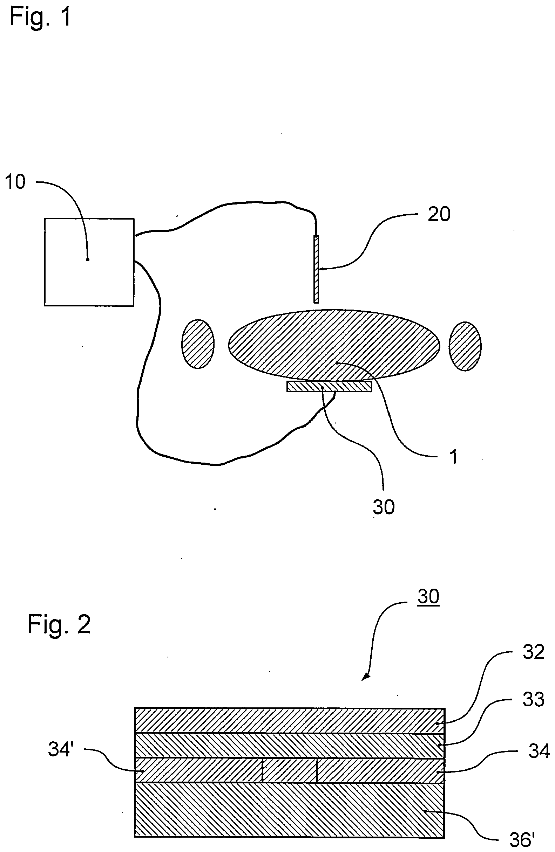

Fig. 1 zeigt ein elektrochirurgisches Gerät. Dieses umfasst einen HF-Generator 10, ein monopolares Instrument 20 und eine Neutralelektrodenanordnung 30. Bei einer Applikation von HF-Strom liegt eine Spannung zwischen dem monopolaren Instrument 20 und der Neutralelektrode 30 an. Der HF-Behandlungsstrom durchfließt den zu behandelnden Körper, im vorliegenden Fall einen Torso 1. In unmittelbarer Nähe zu dem monopolaren Instrument 20 ist die Stromdichte derart hoch, dass das anliegende Gewebe koaguliert oder durchtrennt wird.

Um Verbrennungen an der Neutralelektrode 30 zu vermeiden, soll erfindungsgemäß ein Latentwärmespeicher vorgesehen werden. Wie in den Fig. 2 - 5 gezeigt, umfasst eine Neutralelektrodenanordnung 30 üblicherweise drei Schichten. Dem biologischen Gewebe benachbart befindet sich die Elektrodenschicht, umfassend mehrere elektrisch voneinander getrennte Elektroden 34, 34'. Danach folgt ein PET-Träger 33, der mit einem Trägervlies 32 verklebt ist.

Zur Verbesserung des elektrischen Kontaktschlusses zwischen dem biologischen Gewebe und den Elektroden 34, 34' wird ein Hydrogel 36 aufgetragen.

Im ersten erfindungsgemäßen Ausführungsbeispiel (vgl. Fig. 2) ist der Latentwärmespeicher im Hydrogel 36 enthalten. Die Fig. 2 zeigt ein Hydrogel 36' mit PCM-Bestandteilen. Das PCM ist in Pulverform und mit dem Hydrogel 36 durchmischt.

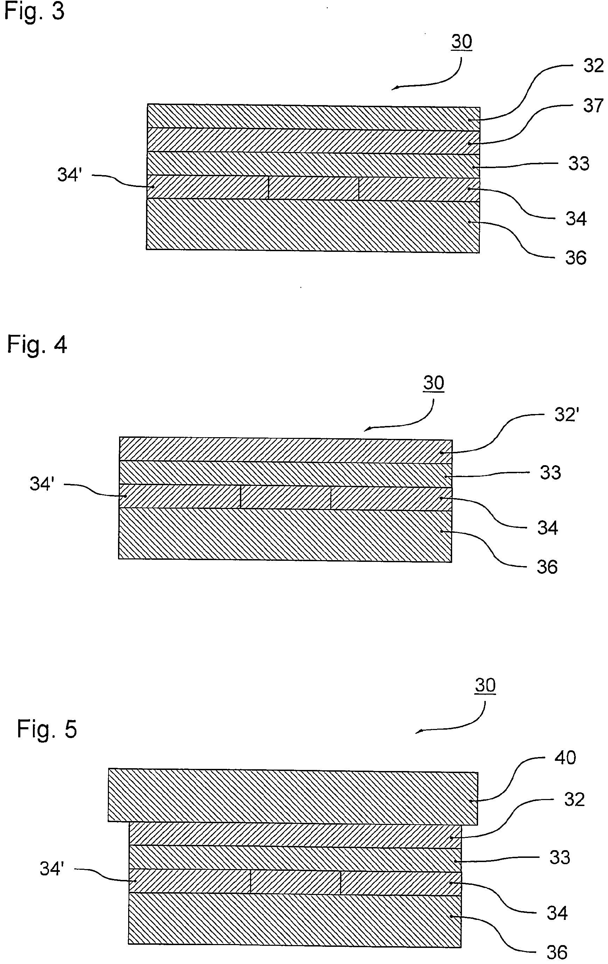

In einem zweiten Ausführungsbeispiel, in Fig. 3, weist die Neutralelektrodenanordnung 30 eine zusätzliche PCM-Schicht 37 auf, die zwischen dem Trägervlies 32 und dem PET-Träger 33 angeordnet ist. Denkbar ist es auch, eine Metalllegierung mit niedrigem Schmelzpunkt zu verarbeiten. Über den PET-Träger 33 kann ein thermisch leitfähiger Kontakt zu den Elektroden 34, 34' hergestellt werden. Die an den Elektroden 34, 34' auftretende Wärme kann somit von der PCM-Schicht 37 absorbiert werden. Alternativ kann auf den PET-Träger verzichtet oder dieser durch PCM ersetzt werden.

In einem dritten Ausführungsbeispiel (vgl. Fig. 4) weist die Neutralelektrodenanordnung 30 ein angepasstes Trägervlies 32 auf. Es handelt sich um ein Trägervlies 32' mit PCM-Fasern. Diese Fasern sind in der Textilindustrie bekannt und können gut als Gewebestruktur verarbeitet werden. Eine unmittelbare Kontaktierung des biologischen Gewebes mit dem PCM wird durch die Verwendung des Trägervlies 32' mit PCM-Fasern vermieden. Somit können an die Verträglichkeit des verwendeten PCM geringere Anforderungen gestellt werden.

In einem vierten Ausführungsbeispiel (vgl. Fig. 5) ist die Neutralelektrodenanordnung 30 um ein PCM-Kissen 40 erweitert. Dieses kann nach der Anbringung der Neutralelektrodenanordnung 30 am biologischen Gewebe großflächig auf die Neutralelektrodenanordnung 30 aufgelegt werden und als Latentwärmespeicher dienen.

Das Kissen ist denkbar einfach in seiner Handhabung und es können große Materialmengen des Phasenübergangsmaterials darin angeordnet werden. Somit hat das PCM-Kissen 40 eine hohe Speicherkapazität.

Vorhergehend wurden einige konkrete Ausführungsbeispiele der Verwendung von PCM in Verbindung mit der Neutralelektrodenanordnung 30 beschrieben. Es ist jedoch auch denkbar, die einzelnen Ausführungsbeispiele miteinander zu verbinden. Beispielsweise kann das Hydrogel 36' mit PCM-Bestandteilen in Verbindung mit dem PCM-Kissen 40 verwendet werden.

### Bezugszeichen

- 1: Torso
- 10: HF-Generator
- 20: monopolares Instrument
- 30: Neutralelektrodenanordnung
- 32: Trägervlies
- 32': Trägervlies mit PCM-Fasern
- 33: PET-Träger
- 34, 34': Elektroden
- 36: Hydrogel
- 36': Hydrogel mit PCM-Bestandteilen
- 37: PCM-Schicht
- 40: PCM-Kissen

## Patentansprüche

1. Neutralelektrodenvorrichtung (30) zur Aufbringung auf die Haut eines Patienten und zur Applikation eines HF-Stroms, umfassend:
- mindestens eine Trägerfaserschicht in Form eines Trägervlieses (32'), wobei die Trägerfaserschicht Phasenübergangsmaterial umfasst, das als Latentwärmespeicher zur Aufnahme von Wärme fungiert;
- Elektroden (34, 34'), wobei der Latentwärmespeicher flächig über die gesamte Elektrodenfläche auf den Elektroden (34, 34') angeordnet ist,
- einen PET-Träger (33) der mit dem Trägervlies verklebt ist, wobei der PET-Träger einen thermisch leitfähigen Kontakt zu den Elektroden (34, 34') herstellt,
wobei
- der Latentwärmespeicher bei der Applikation auf einer dem biologischem Gewebe abgewandten, ersten Seite der Elektrode angeordnet ist,
- Hydrogel (36) auf der anderen, zweiten Seite der Elektrode (34, 34') vorgesehen ist.

2. Neutralelektrodenvorrichtung nach Anspruch 1,
**gekennzeichnet durch**
mindestens eine Elektrode (34, 34') aus Aluminium.

3. Elektrochirurgisches Instrument zur Koagulation und/oder zum Schneiden von Gewebe, umfassend eine Neutralelektrodenvorrichtung (30) nach einem der Ansprüche 1 oder 2.

4. Neutralelektrodenvorrichtung nach einem der Ansprüche 1 oder 2,
**dadurch gekennzeichnet, dass**
das Phasenübergangsmaterial aus der Stoffgruppe der Paraffine ist.

5. Neutralelektrodenvorrichtung nach Anspruch 4,
**dadurch gekennzeichnet, dass**
das Phasenübergangsmaterial in Silikat verkapselt ist.

6. Neutralelektrodenvorrichtung nach einem der Ansprüche 1 bis 5
**dadurch gekennzeichnet, dass**
der Latentwärmespeicher einen Schmelzpunkt aufweist, der niedriger als eine Maximaltemperatur ist, bei der es zur thermischen Beschädigung von biologischem Gewebe kommt.

7. Neutralelektrodenvorrichtung nach Anspruch 6,
**dadurch gekennzeichnet, dass**
die Maximaltemperatur niedriger als 60°C ist.

8. Neutralelektrodenvorrichtung nach Anspruch 6 oder 7,
**dadurch gekennzeichnet, dass**
der Schmelzpunkt höher als eine Minimaltemperatur, nämlich höher als 25°C ist.

## Claims

1. Neutral electrode device (30) for laying onto the skin of a patient and for application of an HF current, comprising:
- at least one support fibre layer in the form of a support nonwoven (32'), the support fibre layer comprising phase-change material which acts as a latent heat store for absorbing heat;
- electrodes (34, 34'), the latent heat store being two-dimensionally arranged on the electrodes (34, 34') over the entire electrode surface,
- a PET support (33) which is adhesively bonded to the support nonwoven, the PET support establishing a thermally conductive contact to the electrodes (34, 34'),
wherein
- the latent heat store during application is arranged on a first side of the electrode that is facing away from the biological tissue,
- hydrogel (36) is provided on the other, second side of the electrode (34, 34').

2. Neutral electrode device according to Claim 1, **characterized by**
at least one electrode (34, 34') composed of aluminium.

3. Electrosurgery instrument for coagulating and/or for cutting tissue, comprising a neutral electrode device (30) according to either of Claims 1 and 2.

4. Neutral electrode device according to either of Claims 1 and 2,
**characterized in that** the phase-change material is from the substance group of the paraffins.

5. Neutral electrode device according to Claim 4, **characterized in that** the phase-change material is encapsulated in silicate.

6. Neutral electrode device according to any of Claims 1 to 5,
**characterized in that** the latent heat store has a melting point which is lower than a maximum temperature at which there is thermal damage of biological tissue.

7. Neutral electrode device according to Claim 6,
**characterized in that** the maximum temperature is lower than 60°C.

8. Neutral electrode device according to Claim 6 or 7,
**characterized in that** the melting point is higher than a minimum temperature, specifically higher than 25°C.

## Revendications

1. Dispositif d'électrode neutre (30) destiné à être appliqué sur la peau d'un patient et destiné à l'application d'un courant HF, comprenant :
- au moins une couche de fibres porteuses sous la forme d'un non-tissé porteur (32'), la couche de fibres porteuses comprenant un matériau à transition de phase qui fait fonction de réservoir de chaleur latente pour réceptionner de la chaleur ;
- des électrodes (34, 34'), le réservoir de chaleur latente étant disposé superficiellement sur toute la surface des électrodes (34, 34') ;
- un support en PET (33) collé avec le non-tissé porteur, le support en PET créant un contact thermiquement conducteur avec les électrodes (34, 34')
où :
- le réservoir de chaleur latente est disposé à l'application sur une première face de l'électrode opposée au tissu biologique,
- un hydrogel (36) est disposé sur l'autre face de l'électrode (34, 34').

2. Dispositif d'électrode neutre selon la revendication 1, **caractérisé en ce qu'**au moins une électrode (34, 34') est en aluminium.

3. Instrument électrochirurgical de coagulation et/ou de découpe des tissus comprenant un dispositif d'électrode neutre (30) selon une des revendications 1 ou 2.

4. Dispositif d'électrode neutre selon une des revendications 1 ou 2, **caractérisé en ce que** le matériau à transition de phase fait partie du groupe des paraffines.

5. Dispositif d'électrode neutre selon la revendication 4, **caractérisé en ce que** le matériau à transition de phase est encapsulé dans un silicate.

6. Dispositif d'électrode neutre selon une des revendications 1 à 5, **caractérisé en ce que** le réservoir de chaleur latente présente un point de fusion inférieur à une température maximale à laquelle des tissus biologiques subissent des dommages thermiques.

7. Dispositif d'électrode neutre selon la revendication 6, **caractérisé en ce que** la température maximale est inférieure à 60 °C.

8. Dispositif d'électrode neutre selon la revendication 6 ou 7, **caractérisé en ce que** le point de fusion est supérieur à une température minimale, à savoir supérieur à 25 °C.
